# EUROPÄISCHE PATENTSCHRIFT

(11) **EP 1 208 918 B1**
(45) Veröffentlichungstag und Bekanntmachung des Hinweises auf die Patenterteilung: **02.05.2003**
(21) Anmeldenummer: 00120858.6
(22) Anmeldetag: 25.09.2000
(51) Int. Cl.: B05C 17/005, A61C 9/00

(54) **Anordnung zum verhältnisgleichen Ausbringen von zwei fliessfähigen Substanzen, insbesondere für Dentalzwecke**
Device for discharging two substances at constant proportion, particularly for dental purposes
Dispositif de décharge à proportion constante de deux substances fluides,en particulier dans le domaine dentaire

(43) Veröffentlichungstag der Anmeldung: 29.05.2002
(73) Patentinhaber: Ernst Mühlbauer GmbH & Co.KG, 25870 Norderfriedrichskoog (DE)
(72) Erfinder: Mühlbauer, Wolfgang, Dr., 22609 Hamburg (DE); Hörth, Hans, 21147 Hamburg (DE); Röbel, Frauke, 22419 Hamburg (DE)
(74) Vertreter: Glawe, Delfs, Moll & Partner

(56) Entgegenhaltungen:
- US-A- 4 260 077
- US-A- 5 104 375

## Beschreibung

Wenn man eine Portion eines Gemischs von zwei fließfähigen Komponenten herstellen will, die zueinander in einem bestimmten Mengenverhältnis stehen müssen, kann man sich zweier Kolben-Zylinder-Spritzen bedienen, die miteinander zu gemeinsamer Bewegung ihrer Kolben gekuppelt sind. Das Mengenverhältnis der Komponenten wird dann durch das Querschnittsverhältnis der Kolben bestimmt. Ein wichtiges Anwendungsgebiet solcher gekuppelter Spritzen ist die Dentaltechnik, nämlich das Anmischen von Mehrkomponentenharzen als Füllmittel oder von Abdruckmasse. Bekannte Anordnungen zum verhältnisgleichen Ausgeben fließfähiger Substanzen umfassen ein Gerät, in welchem die Kolben beider Spritzen synchron vorgeschoben werden (EP-B 492413, EP-A 1010401, WO 9105731). Darin sind einerseits die Spritzenkörper starr nebeneinander gehalten und andererseits die Kolben über miteinander verbundene Schubstangen ebenfalls starr miteinander verbunden, wobei sie jeweils die gleiche Stellung in den zugehörigen Spritzenkörpern haben. Demnach muß auch der Entleerungszustand beider Spritzenkörper stets übereinstimmen. Dies ist dann unproblematisch, wenn eine Komponente stets mit derselben anderen Komponente verwendet wird und demzufolge die sie enthaltenden Spritzen nur gemeinsam benutzt werden. Jedoch gibt es Fälle, in denen diese Voraussetzung nicht erfüllt ist. Beispielsweise gibt es Dentalharze, die in der Regel ohne eine zweite Komponente verwendet und dann mittels Strahlung gehärtet werden, die aber ausnahmsweise auch in Mischung mit einem Härter als zweiter Komponente benutzt werden. In diesen Fällen ist man bislang darauf angewiesen, die Komponenten einzeln zu dosieren und von Hand zu mischen. Es sind auch Systeme bekannt, in denen zwei Spritzen die einander zugeordneten Komponenten enthalten, getrennt gefertigt und von Fall zu Fall für die Applikation in einem Gerät zusammengeführt werden (WO9105731, US 4 260 077 A, US 5 104 375 A).

Voraussetzung ist dabei ein jeweils übereinstimmender Füllzustand.

Es wäre zweckmäßig, für die gleichbleibende Grundkomponente, die variierend von Fall zu Fall mit oder ohne Zusatzkomponente verwendet werden soll, stets dieselbe Spritze verwenden zu können, während die Spritzen für die Zusatzkomponenten beliebig und ohne Rücksicht auf ihren zufälligen Füllzustand damit kombiniert werden können. Der Erfindung liegt die Aufgabe zugrunde, eine Anordnung zu schaffen, die dies gestattet. Die Lösung liegt in den Merkmalen des Anspruchs 1 sowie vorzugsweise in denjenigen der Unteransprüche.

Danach ist vorgesehen, daß sowohl die Spritzenkörper als auch die Spritzenkolben unabhängig von der jeweiligen Kolbenstellung in Vorschubrichtung starr durch lösbare Kupplungseinrichtungen miteinander gekuppelt werden können. Die Kupplungseinrichtungen sind so ausgebildet, daß die Kolben und/oder die Spritzenkörper in beliebiger Relativstellung zueinander (in Vorschubrichtung) verbunden werden können. Bevorzugt wird eine Anordnung, bei welcher die Spritzenkörper nur in einer vorbestimmten Relativstellung, die den Kolben zugehörigen Kolbenstangen aber in einer beliebigen relativen Stellung miteinander kuppelbar sind.

Die Einrichtungen zum Kuppeln der Spritzenkörper miteinander können gesondert von diesen vorgesehen sein. Zweckmäßiger ist es aber, die Spritzenkörper selbst mit zueinander passenden Kupplungseinrichtungen zu versehen. Sie sind zweckmäßigerweise fest mit wenigstens einem von ihnen verbunden oder bilden einen festen Bestandteil davon.

Die Spritzenkörper sind an ihrem vorderen Ende meist mit einem Ausbringstutzen versehen. Wenn zwei Komponenten gemeinsam ausgebracht werden, sollen sie in der Regel zusammen verarbeitet, ggfs. sogar aus einer Mischeinrichtung gemeinsam ausgegeben werden. Es ist deshalb zweckmäßig, die Kupplungseinrichtung für die Spritzenkörper so auszubilden, daß sich deren Ausbringstutzen nahe beieinander oder wenigstens in definierter Stellung zueinander befinden. Um dies in Vorschubrichtung sicherzustellen, kann erfindungsgemäß vorgesehen sein, daß ein Teil der an einem der beiden Spritzenkörper vorgesehenen oder anzubringenden Teil der Kupplungseinrichtung als Anschlag für eine Frontfläche des anderen Spritzenkörpers entgegen der Vorschubrichtung ausgebildet ist. Um dies quer zur Vorschubrichtung sicherzustellen, kann die Einrichtung zum Kuppeln der Spritzenkörper so ausgebildet sein, daß der Ausbringstutzen wenigstens eines der beiden Spritzenkörper quer zur Vorschubrichtung dadurch lokalisiert wird. Der Querabstand der Ausbringstutzen ist insbesondere dann wichtig, wenn auf die Stutzen eine Düse zum gemeinsamen Ausbringen und ggfs. Mischen der beiden Substanzen aufgesetzt werden soll. Nach der Erfindung kann die Kupplungseinrichtung mit einer Einrichtung zum lösbaren Halten einer solchen Düse versehen sein. Für die relative Festlegung der Spritzenkörper in Vorschubrichtung kann der erwähnte eine Anschlag genügen. Zweckmäßigerweise ist aber zusätzlich oder statt dessen noch ein zweiter Anschlag vorgesehen, der den durch den Frontanschlag in einer Richtung festgelegten Spritzenkörper auch noch in der anderen Richtung sichert. Dies geschieht zweckmäßigerweise durch einen weiter hinten gelegenen Teil der Kupplungseinrichtung.

Damit die Spritzenkörper in der gekuppelten Stellung verbleiben, sind zweckmäßigerweise geeignete Fixiermittel vorgesehen, die beispielsweise von einer Schnapphalterung gebildet sind.

Die Spritzenkörper werden zweckmäßigerweise parallel zueinander gekuppelt, damit die miteinander gekuppelten Abschnitte der Kolbenstangen während ihres Vorschubs konstanten Abstand voneinander behalten.

Die Einrichtung zum Kuppeln der Kolbenstangen kann fest am Ende einer der beiden Kolbenstangen angeordnet sein. Das bedeutet, daß die andere Kolbenstange etwa doppelt so lang sein muß, damit eine wirksame Kupplungsverbindung auch dann noch geschlossen werden kann, wenn die mit der Kupplungseinrichtung für die Kolbenstangen versehene Spritze noch voll, die andere aber nahezu leer ist. Dies gilt nicht, wenn eine gesonderte Kupplung verwendet wird oder wenn beide Kolbenstangenenden eine Kupplungseinrichtung tragen.

Die Kupplungseinrichtung für die Kolbenstangen ist zweckmäßigerweise als Klammer ausgebildet, um unter der Kraft einer sie schließenden Feder die betreffende Kolbenstange so fest zu umschließen, daß eine hinreichende Reibung zur Mitnahme in Vorschubrichtung erzeugt wird. Es können aber auch Schließeinrichtungen daran vorgesehen sein, die diese Kupplungskraft erzeugen.

Um einen sicheren Griff der Klammer an der Kolbenstange zu ermöglichen, sind zweckmäßigerweise die Oberflächen beider ineinander eingreifend ausgebildet. Dafür genügt bereits eine Aufrauhung, deren Rauhigkeitserhöhungen statistisch in Rauhigkeitsvertiefungen der jeweils anderen Fläche eingreifen. Die Oberflächen können aber auch regelmäßig gerastert oder (quer zur Vorschubrichtung) gerippt sein. Solche Strukturen sollen so fein sein, daß sie beim Anlegen der Klammer nicht zu einer wesentlichen Veränderung der vorhandenen Kolbenstände führen.

Die Erfindung wird im folgenden näher unter Bezugnahme auf die Zeichnung erläutert, die vorteilhafte Ausführungsbeispiele veranschaulicht. Es zeigen:
- Fig. 1: eine erste Spritze zur alleinigen Verwendung,
- Fig. 2: die erste Spritze zusammengesetzt mit einer zweiten Spritze,
- Fig. 3: eine Zerlegungszeichnung der ersten und der zweiten Spritze,
- Fig. 4 und 5: eine Kupplungseinrichtung für Kolbenstangen in zwei unterschiedlichen Funktionsstellungen,
- Fig. 6 bis 8: eine weitere Ausführungsform einer Klammer zum Verbinden von Kolbenstangen,
- Fig. 9: eine weitere Ausführungsform zum Verbinden zweier Kolbenstangen und
- Fig. 10 bis 12: eine weitere Ausführungsform einer Einrichtung zum Verbinden von zwei Kolbenstangen.

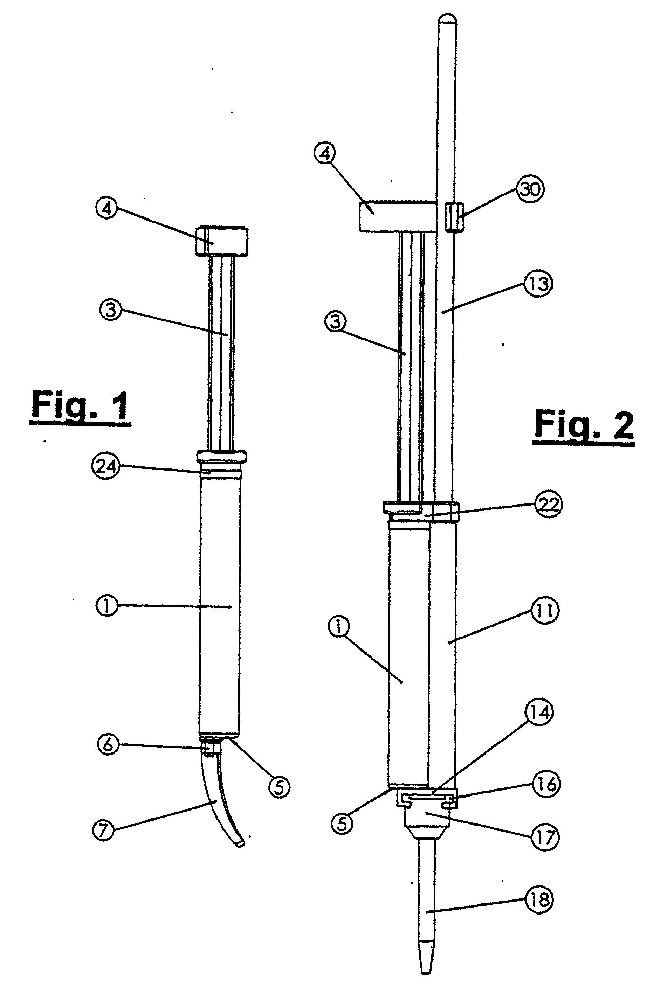

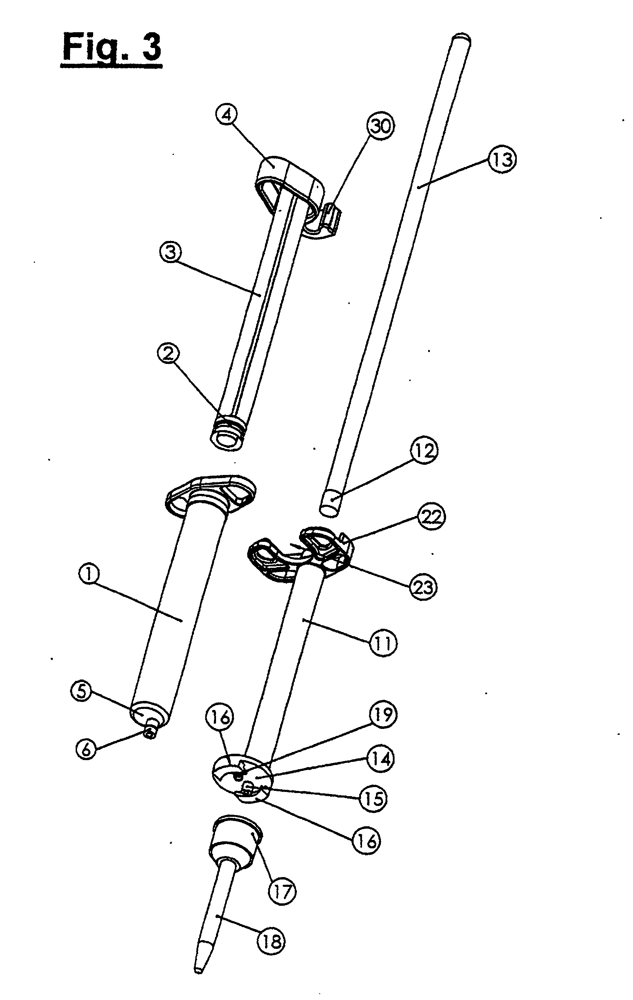

Figur 1 bis 3 zeigen eine erste Spritze mit Spritzenkörper 1, Kolben 2, Kolbenstange 3 und Kolbenstangenkopf 4. Der Spritzenkörper besteht aus einem Hohlzylinder, der am vorderen Ende durch eine Frontplatte 5 geschlossen ist, von der sich ein Ausbringstutzen 6 erhebt. Wenn die erste Spritze allein verwendet wird, wird auf den Ausbringstutzen 6 eine Düse 7 aufgesetzt.

Gemäß Figur 2 ist die erste Spritze zusammengesetzt mit einer zweiten Spritze mit einem Spritzenkörper 11, einem Kolben 12 und einer damit verbundenen Kolbenstange 13, die etwa doppelt so lang ist wie die Kolbenstange 3. Der Kolben braucht sich im Durchmesser von der Kolbenstange nicht zu unterscheiden. Er kann beispielsweise von einem Dichtring oder einer Dichtkante gebildet sein. Im einfachsten Fall wird der Kolben von der Stirnfläche und Stirnkante einer ungegliederten Stange konstanten Durchmessers gebildet.

Am vorderen Ende des Spritzenkörpers 11 befindet sich eine Anschlagplatte 14, aus der unterseitig der dem Spritzenkörper 11 zugehörige Ausbringstutzen 15 hervorsteht. Außerdem trägt die Anschlagplatte 14 vorderseitig Halterungen 16 für den Kopf 17 einer Ausbringdüse 18.

Der Ausbringstutzen 15 sitzt exzentrisch in der Anschlagplatte 14 in bezug auf die Halterungen 16. Symmetrisch zum Ausbringstutzen 15 ist in der Anschlagplatte 14 eine Bohrung 19 enthalten, deren Durchmesser nicht oder wenig größer ist als der Außendurchmesser des Ausbringstutzens 6 der ersten Spritze. Beim Ansetzen des Spritzenkörpers 1 der ersten Spritze an den Spritzenkörper 11 der zweiten Spritze steckt man den Ausbringstutzen 6 des ersten Spritzenkörpers 1 durch die Bohrung 19, so daß er parallel zu dem Ausbringstutzen 15 der zweiten Spritze aus der Bohrung herausragt. Der Kopf 17 der Düse 18 ist so ausgebildet, daß er in bekannter, hier nicht interessierender Weise mit beiden Ausbringstutzen 6, 15 verbunden und in der verbundenen Stellung durch die Halterungen 16 fixiert werden kann.

Wenn der Ausbringstutzen 6 des Spritzenkörpers 1 durch die Bohrung 19 gesteckt ist, liegt die Stirnfläche 5 des Spritzenkörpers 1 an der Rückfläche der Anschlagplatte 14 an, wie dies in Figur 2 gezeigt ist. Die axiale Stellung des Spritzenkörpers 1 wird dann durch das Zusammenwirken der Stirnfläche 5 mit der Rückfläche der Anschlagplatte 14 bestimmt.

Am hinteren Ende des Spritzenkörpers 11 ist eine Gabelplatte 22 vorgesehen, deren Arme einen Zwischenraum 23 einschließen, der zum Durchmesser des Spritzenkörpers 1 paßt. Genauer gesagt, paßt die Weite des Zwischenraums 23 zu dem Durchmesser einer Ringnut 24 am hinteren Ende des Spritzenkörpers 1, deren axiale Länge der axialen Dicke der Arme der Gabelplatte 22 entspricht. Wenn man nach dem Einsetzen des Ausbringstutzens 6 in die Bohrung 19 den Spritzenkörper 1 an den Spritzenkörper 11 heranschwenkt, treten die Arme der Armplatte 22 in die Haltenut 24 ein und bilden mit deren Flanken zusammenwirkende Anschläge, die - ggfs. gemeinsam mit der Anschlagplatte 14 - die axiale Stellung des Spritzenkörpers 1 gegenüber dem Spritzenkörper 11 in enger Toleranz bestimmen.

Damit die Spritzenkörper in dieser Stellung verharren, können die Arme der Gabelplatte 22 als Schnapphalterung ausgebildet sein, so daß der Spritzenkörper 1 die gekuppelte Stellung nur unter Überwindung einer Kraftschwelle verlassen kann. Jedoch ist dies dann mitunter nicht notwendig, wenn die Verbindung der Kupplungsstangen die Spritzen in der gewünschten Lage sichert.

Es versteht sich, daß die im Beispiel hinten an den Spritzenkörpern vorgesehene Schnapphalterung auch vorne angeordnet sein kann oder beide Enden der Spritzenkörper mit Schnapphalterungen versehen sein können. Auch ist es möglich, die Einrichtung zur Verbindung einer Ausbring- oder Mischdüse mit den Spritzenkörpern zum Kuppeln derselben zu verwenden. Beispielsweise kann eine Überwurfhalterung verwendet werden, die beide Spritzenkörper erfaßt, indem einander ergänzende (beispielsweise hälftig gleiche) Teile der Spritzenkörper das Gegenstück zu der Überwurfhalterung bilden. Die Überwurfhalterung kann (im Gegensatz zu den Spritzen und Düsen) als wiederverwendbares Teil beispielsweise aus Metall konzipiert sein.

Der Kolbenstangenkopf 4 der ersten Spritze ist einerseits so ausgebildet, daß er in bekannter Weise als Druckplatte zur alleinigen Benutzung dieser Spritze geeignet ist. Andererseits ist er mit einem Haken 30 versehen, dessen Mittenabstand zur Kolbenstange 3 dem Mittenabstand der gekuppelten Spritzenkörper 1, 11 gleicht. Der Haken 30 ist als Klammer zur Aufnahme der Kolbenstange 13 ausgebildet, die sich nach geringer elastischer Verformung des Hakens in den Hakenraum einschnappen läßt. Die Kraft, mit der der Haken 30 die Kolbenstange 13 umklammert, ist so groß, daß die dadurch in Vorschubrichtung erzeugte Reibung zwischen diesen Teilen größer ist als der Widerstand, den der Kolben 12 beim Ausbringen der im Spritzenkörper 11 enthaltenen Masse vorfindet. Das bedeutet, daß beim Druck auf den Kolbenstangenkopf 4 beide Kolben starr miteinander verbunden vorgeschoben werden. Da der Haken 30 an jeder axialen Stelle mit der Kolbenstange 13 verhakt werden kann, ist die Kupplung der Kolben unabhängig von ihrer jeweiligen Vorschubstellung. Es muß lediglich dafür gesorgt werden, daß beim Schließen der Kupplungsverbindungen zwischen dem Haken 30 und der Kolbenstange 13 beide Kolben zwischenraumfrei an der auszuschiebenden Masse anliegen.

Will man die erste Spritze wieder allein oder in Verbindung mit einer anderen zweiten Spritze verwenden, so kann man die Kupplungseinrichtungen ohne weiteres wieder lösen.

Damit der Haken 30 nicht an der Kolbenstange 13 verrutscht, ist die Oberfläche der Kolbenstange 13 und/oder die Innenfläche des Hakens 30 zweckmäßigerweise aufgerauht oder quer geriffelt oder gerippt.

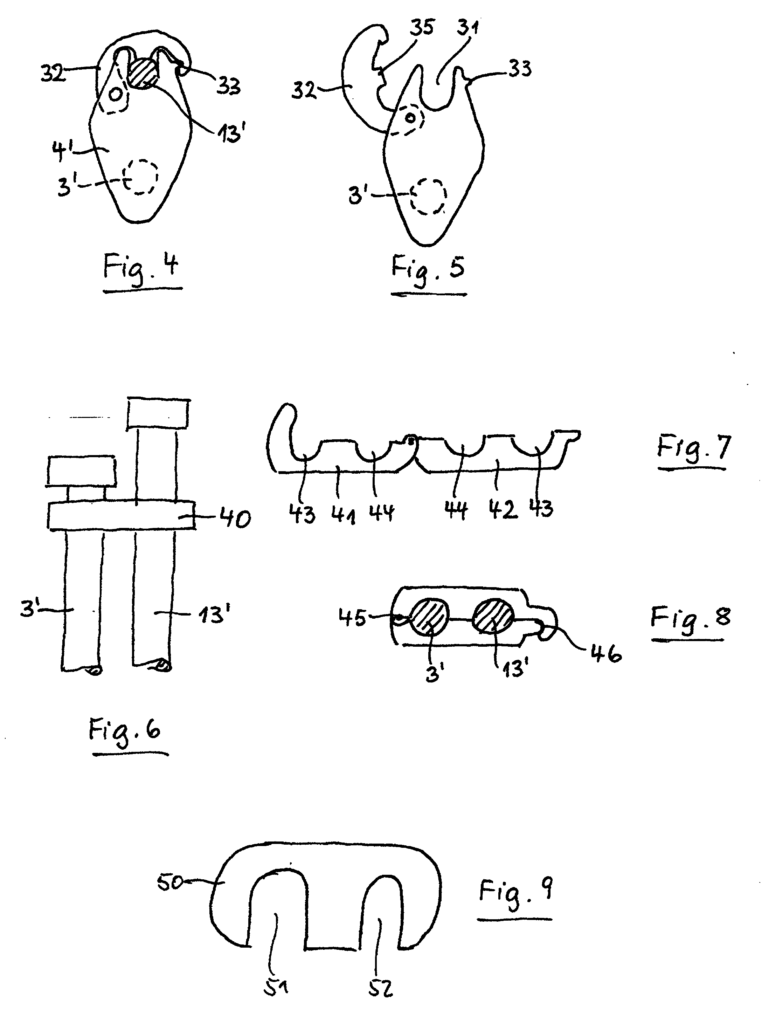

Es versteht sich, daß die von dem Haken 30 gebildete elastische Klammer durch andere Bauformen ersetzt werden kann, die derselben Funktion dienen. Beispielsweise kann man, falls die Klemmkraft des Hakens 30 nicht ausreicht, ihn mit einer zusätzlichen Klemmeinrichtung ausbilden, wie dies in Figur 4 und 5 gezeigt ist. Statt des Hakens 30 ist an dem Kopf 4' der Kolbenstange 3' eine gabelförmige Aufnahme 31 für die Kolbenstange 13 vorhanden, die durch einen Bügel 32 geschlossen werden kann, der mittels eines Schnappverschlusses 33 in der geschlossenen Stellung gehalten wird und in dieser Stellung durch einen Bügelteil 35 den Druck auf die Kolbenstange 13 verstärkt und/oder die Gabelteile, die den Raum 31 begrenzen, zusammendrückt.

Auf die gegenüber der Kolbenstange 3 vergrößerte Länge der Kolbenstange 13 kann man verzichten, wenn man zur Verbindung der Kolbenstangen eine besondere Klammer verwendet, wie dies in Figur 6 bis 8 dargestellt ist. Für die Verbindung der Kolbenstangen 3',13' ist die Klammer 40 vorgesehen, die aus zwei Klapphälften 41, 42 besteht, die miteinander Aufnahmeöffnungen 43, 44 für je eine Kolbenstange 3', 13' einschließen, wenn sie geschlossen sind. Sie sind an der einen Seite mittels eines Scharniers 45 und am anderen Ende mittels eines Schnappverschlusses 46 miteinander verbunden.

Ein sehr einfaches Kupplungsglied zeigt Figur 9, nämlich eine Scheibe 50 mit zwei parallelen, U-förmigen Ausschnitten 51, 52, deren Breite jeweils einer Kolbenstange so angepaßt ist, daß sie von der Seite her unter elastischer Verformung und entsprechender Klemmspannung an beliebiger Stelle auf die Kolbenstangen aufgeschoben werden können.

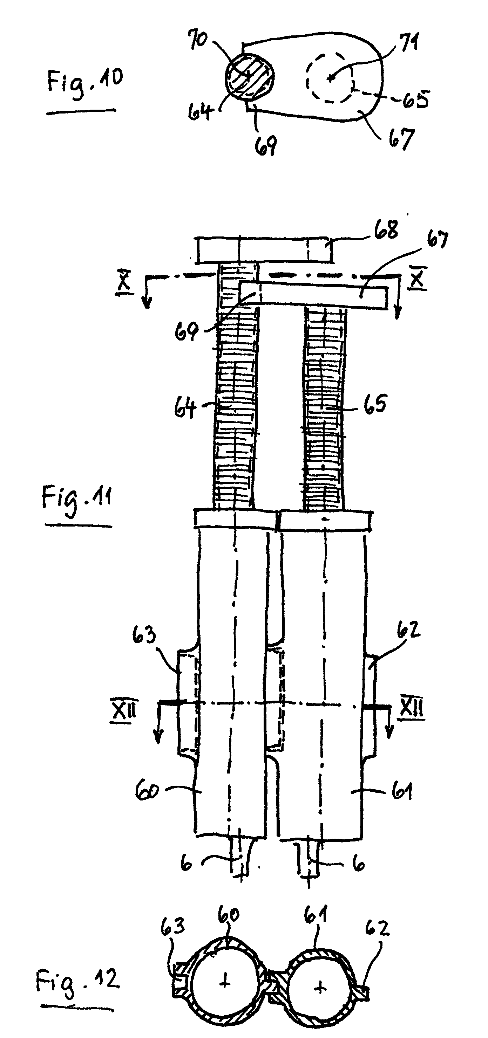

Schließlich zeigen die Figuren 10 bis 12 eine letzte Ausführungsform von Kupplungseinrichtungen. Jeder Spritzenkörper 60, 61 ist auf einer Seite mit einer Rippe 62 und auf der anderen Seite mit einer dazu passenden Nut 63, die von vorstehenden Nutflanken gebildet wird, versehen. Den Querschnitt veranschaulicht Fig. 12 (Schnitt gemäß Linie XII-XII). Die Kolbenstangen 64, 65, die in Umfangsrichtung geriffelt sind, tragen Kolbenstangenköpfe 67, 68, die die in Figur 10 (Schnitt gemäß Linie X-X) dargestellte Form haben. Sie weisen einen einseitigen Vorsprung auf, der am Ende eine halbkreisförmige Ausnehmung 69 trägt, deren Mittelpunkt 70 von der Mittelachse 71 der zugehörigen Kolbenstange 65 ebenso weit entfernt ist, wie die Mittelachsen der Kolbenstangen 64, 65 voneinander oder etwas mehr. Die Oberfläche der Ausnehmung 69 ist in der gleichen Weise geriffelt wie die Kolbenstangen 64, 65. Wenn man die beiden Spritzenkörper 60, 61 miteinander kuppelt, sorgt man dafür, daß der seinem Spritzenkörper jeweils nächste Kolbenstangenkopf 67 der benachbarten Kolbenstange 64 zugewendet ist, so daß diese von der Ausnehmung 69 aufgenommen wird und mit dieser formschlüssig in Vorschubrichtung zusammenwirkt. Während der Benutzung hält man die Spritzenkörper in solcher Weise, daß die Kupplungsverbindung sowohl zwischen den Spritzenkörpern als auch den Kolbenstangen aufrechterhalten wird.

Zur Verbindung der Spritzenkörper und/oder der Kolbenstangen kann auch ein Klettverschluß verwendet werden.

Die Erfindung erlaubt es, eine Spritze wahlweise mit oder ohne verhältnisgleich zu betreibende Zusatzspritzen zu verwenden, ohne daß es auf gleichen Füllungsgrad ankommt. So können beispielsweise Dentalmaterialien je nach der Art der Zusatzkomponente mit unterschiedlichen Härtungsmechanismen, unterschiedlichen Konsistenzen, unterschiedlichen Erhärtungszeiten oder unterschiedlicher Farbgebung ausgestattet werden. Auch kann die Zusatzkomponente Wirkstoffe enthalten, die das Anwendungsgebiet variieren, z.B. Antibiotika, Säuren oder fluoridfreisetzende Füllstoffe. Auch die Konzentration eines Wirkstoffs kann auf diese Weise variiert werden.

Die Erfindung ist nicht auf die gemeinsame Verwendung von zwei Spritzen beschränkt. Es können in derselben Weise drei oder mehr Spritzen zu synchroner Betätigung miteinander verbunden werden.

## Patentansprüche

1. Anordnung zum verhältnisgleichen Ausbringen von zwei oder mehr fließfähigen Substanzen aus zwei oder mehr Spritzen, von denen zumindest eine auch alleine oder in Verbindung mit anderen Spritzen benutzt wird, insbesondere für Dentalzwecke, weiche Anordnung lösbare Einrichtungen (5, 14, 22, 24, 30, 31, 40, 50, 62, 63, 67, 68) zum in Vorschubrichtung starren Kuppeln der Spritzenkörper (1, 11, 60, 61) sowie der Spritzenkolben (2, 12) aufweist, Kolben (2, 12) oder deren Kolbenstangen (3, 13, 3', 13', 64 ,65) unabhängig von der jeweiligen Kolbenstellung in beliebiger relativer Stellung in Vorschubrichtung miteinander kuppelbar sind.

2. Anordnung nach Anspruch 1, **dadurch gekennzeichnet, daß** die Spritzenkörper (1, 11, 60, 61) nur in einer vorbestimmten relativen Stellung in Vorschubrichtung miteinander kuppelbar sind.

3. Anordnung nach Anspruch 2, **dadurch gekennzeichnet, daß** die Einrichtung (5, 14, 24, 22, 62, 63) zum Kuppeln der Spritzenkörper (1, 11, 60, 61) fest mit wenigstens einem von ihnen verbunden ist.

4. Anordnung nach einem der Ansprüche 1 bis 3, **dadurch gekennzeichnet, daß** ein Teil (14) der Einrichtung zum Kuppeln der Spritzenkörper (1, 11) als Anschlag für eine Frontfläche (5) des Spritzenkörpers (1) entgegen der Vorschubrichtung ausgebildet ist.

5. Anordnung nach einem der Ansprüche 2 bis 4, dadurch gekennzeichent, daß die Einrichtung (14, 19) zum Kuppeln der Spritzenkörper (1, 11) auch zum Lokalisieren des Ausbringstutzens (6) wenigstens eines der beiden Spritzenkörper (1, 11) quer zur Vorschubrichtung ausgebildet ist.

6. Anordnung nach Anspruch 5, **dadurch gekennzeichnet, daß** der wenigstens einen Ausbringstutzen (6) lokalisierende Teil (14) der Kupplungseinrichtung mit einer Düse (18) zum gemeinsamen Ausbringen und ggfs. Mischen der beiden Substanzen oder einer Einrichtung (16) zum lösbaren Halten einer solchen Düse (18) versehen ist.

7. Anordnung nach Anspruch 6, **dadurch gekennzeichnet, daß** die Einrichtung (14, 22) zum Kuppeln zweier Spritzenkörper (1, 11) außer den einen Anschlag (5, 14) entgegen der Vorschubrichtung bildenden Teilen einen weiter hinten gelegenen Teil (22, 24) aufweist, der einen gegensinnigen Anschlag bildet.

8. Anordnung nach Anspruch 7, **dadurch gekennzeichnet, daß** der weiter hinten gelegene Teil (22, 24) der Kupplungseinrichtung mit einer quer zur Vorschubrichtung wirkenden Schnapphalterung versehen ist.

9. Anordnung nach einem der Ansprüche 1 bis 8, **dadurch gekennzeichnet, daß** die Einrichtung zum Kuppeln der Spritzenkolben als Einrichtung (30, 33, 40, 50, 67, 68) zum Kuppeln von mit den Kolben (2, 12) verbundenen Kolbenstangen (3, 13, 3', 13', 64, 65) ausgebildet ist.

10. Anordnung nach Anspruch 9, **dadurch gekennzeichnet, daß** mit einer Kolbenstange (3, 3') eine Klammer (30, 31) in Vorschubrichtung fest zum Erfassen der anderen Kolbenstange (13, 13') verbunden ist.

11. Anordnung nach Anspruch 9, **dadurch gekennzeichnet, daß** eine gesonderte Klammer (40, 50) zum Verbinden beider Kolbenstangen (3, 3', 13, 13') vorgesehen ist.

12. Anordnung nach Anspruch 10 oder 11, **dadurch gekennzeichnet, daß** die Kolbenstangen (3, 3', 13, 13', 64, 65) und die damit zusammenwirkenden Flächen der Klammern (30, 31, 40, 50, 69) ineinander eingreifend ausgebildet sind.

## Claims

1. System for the release of equal proportions of two or more flowable substances from two or more syringes, of which at least one is also used alone or in combination with other syringes, especially for dental purposes, which system comprises detachable devices (5, 14, 22, 24, 30, 31, 40, 50, 62, 63, 67, 68) for coupling the syringe bodies (1, 11, 60, 61) and the syringe pistons (2, 12) rigidly in the direction of advance, **characterized in that** the pistons (2, 12) or their piston rods (3, 13, 3', 13', 64, 65) can be coupled to one another in the direction of advance in any desired relative position irrespective of the current position of the piston.

2. System according to Claim 1, **characterized in that** the syringe bodies (1, 11, 60, 61) can be coupled to one another in the direction of advance only in a predetermined relative position.

3. System according to Claim 2, **characterized in that** the device (5, 14, 24, 22, 62, 63) for coupling the syringe bodies (1, 11, 60, 61) is fixedly connected to at least one thereof.

4. System according to one of Claims 1 to 3, **characterized in that** a part (14) of the device for coupling the syringe bodies (1, 11) is formed as a stop for a frontal surface (5) of the syringe body (1) counter to the direction of advance.

5. System according to one of Claims 2 to 4, **characterized in that** the device (14, 19) for coupling the syringe bodies (1, 11) is also formed to localize the release pipe (6) of at least one of the two syringe bodies (1, 11) transversely to the direction of advance.

6. System according to Claim 5, **characterized in that** the part (14) of the coupling device localizing at least one release pipe (6) is provided with a nozzle (18) for the joint release and optional mixing of the two substances or a device (16) for the releasable retention of such a nozzle (18).

7. System according to Claim 6, **characterized in that** the device (14, 22) for coupling two syringe bodies (1, 11) comprises, in addition to the parts forming a stop (5, 14) counter to the direction of advance, a more rearward part (22, 24) which forms a stop in the opposite direction.

8. System according to Claim 7, **characterized in that** the more rearward part (22, 24) of the coupling device is provided with a snap-fit mounting acting transversely to the direction of advance.

9. System according to one of Claims 1 to 8, **characterized in that** the device for coupling the syringe pistons is formed as a device (30, 33, 40, 50, 67, 68) for coupling piston rods (3, 13, 3', 13', 64, 65) connected to the pistons (2, 12).

10. System according to Claim 9, **characterized in that** a clamp (30, 31) is fixedly connected to a piston rod (3, 3') in the direction of advance to grip the other piston rod (13, 13').

11. System according to Claim 9, **characterized in that** a separate clamp (40, 50) is provided to connect the two piston rods (3, 3', 13, 13').

12. System according to Claim 10 or 11, **characterized in that** the piston rods (3, 13, 3', 13', 64, 65) and the surfaces of the clamps (30, 31, 40, 50, 69) interacting therewith are formed to engage into one another.

## Revendications

1. Agencement pour éjecter de manière proportionnée deux ou plusieurs substances fluides hors de deux ou plusieurs seringues, parmi lesquelles au moins une est utilisée également seule ou en combinaison avec d'autres seringues, en particulier à des fins dentaires, lequel agencement comporte des dispositifs amovibles (5, 14, 22, 24, 30, 31, 40, 50, 62, 63, 67, 68) pour coupler de manière rigide dans le sens d'avance, les corps de seringue (1, 11, 60, 61), ainsi que les pistons de seringue (2, 12), **caractérisé en ce que** les pistons (2, 12) ou les tiges de piston (3, 13, 3', 13', 64, 65), indépendamment de la position de chaque piston, peuvent être couplés les uns avec les autres dans une position relative quelconque dans le sens d'avance.

2. Agencement selon la revendication 1, **caractérisé en ce que** les corps de seringue (1, 11, 60, 61) peuvent être couplés les uns avec les autres uniquement dans une position relative prédéterminée dans le sens d'avance.

3. Agencement selon la revendication 2, **caractérisé en ce que** le dispositif (5, 14, 24, 22, 62, 63) pour coupler les corps de seringue (1, 11, 60, 61) est assemblé de manière inamovible avec au moins l'un d'entre eux.

4. Agencement selon l'une des revendications 1 à 3, **caractérisé en ce qu'**une partie (14) du dispositif pour coupler les corps de seringue (1, 11) est conçue sous forme de butée pour une surface frontale (5) du corps de seringue (1) dans le sens opposé au sens d'avance.

5. Agencement selon l'une des revendications 2 à 4, **caractérisé en ce que** le dispositif (14, 19) pour coupler les corps de seringue (1, 11) est aussi conçu pour positionner le manchon d'injection (6) d'au moins un des deux corps de seringue (1, 11) transversalement au sens d'avance.

6. Agencement selon la revendication 5, **caractérisé en ce que** la partie (14) du dispositif de couplage, destinée à positionner au moins un manchon d'injection (6), est munie d'une buse (18) pour l'injection commune ou, le cas échéant, le mélange des deux substances ou d'un dispositif (16) pour la fixation amovible d'une telle buse (18).

7. Agencement selon la revendication 6, **caractérisé en ce que** le dispositif (14, 22) pour coupler deux corps de seringue (1, 11) comporte, outre les parties formant une butée (5, 14) dans le sens opposé au sens d'avance, une autre partie (22, 24) située plus en arrière qui forme une butée en sens inverse.

8. Agencement selon la revendication 7, **caractérisé en ce que** la partie (22, 24), située plus en arrière, du dispositif de couplage est munie d'une fixation à encliqueter agissant transversalement au sens d'avance.

9. Agencement selon une des revendications 1 à 8, **caractérisé en ce que** le dispositif pour coupler les pistons de seringue est conçu sous forme de dispositif (30, 33, 40, 50, 67, 68) pour coupler des tiges de piston (3, 13, 3', 13', 64, 65) liées aux pistons (2, 12).

10. Agencement selon la revendication 9, **caractérisé en ce qu'**un crochet (30, 31) est assemblé de manière inamovible dans le sens d'avance avec une tige de piston (3, 3') en vue de saisir l'autre tige de piston (13, 13').

11. Agencement selon la revendication 9, **caractérisé en ce qu'**un crochet (40, 50) séparé est prévu pour assembler les deux tiges de piston (3, 3', 13, 13').

12. Agencement selon la revendication 10 ou 11, **caractérisé en ce que** les tiges de piston (3, 3', 13, 13', 64, 65) et les surfaces des crochets (30, 31, 40, 50, 69) coopérant avec celles-ci sont conçues pour entrer en prise les unes avec les autres.
